**Europäisches Patentamt**

**European Patent Office**

**Office européen des brevets**

(19)

(11) Veröffentlichungsnummer: **0 224 684 B1**

(12) **EUROPÄISCHE PATENTSCHRIFT**

(45) Veröffentlichungstag der Patentschrift:
**25.01.89**

(51) Int. Cl.⁴: **C07D 307/12**

(21) Anmeldenummer: **86114054.9**

(22) Anmeldetag: **10.10.86**

(54) Verfahren zur Herstellung von Tetrahydrofurfuryl-(2)-methacrylat und dazu geeigneter Katalysator.

(30) Priorität: **06.12.85 DE 3543115**

(43) Veröffentlichungstag der Anmeldung:
**10.06.87 Patentblatt 87/24**

(45) Bekanntmachung des Hinweises auf die Patenterteilung:
**25.01.89 Patentblatt 89/4**

(84) Benannte Vertragsstaaten:
**AT BE CH DE FR GB IT LI NL SE**

(56) Entgegenhaltungen:
**DE-A- 3 423 441**
**DE-A- 3 423 443**

(73) Patentinhaber: **Degussa Aktiengesellschaft,
Weissfrauenstrasse 9, D-6000 Frankfurt am Main 1(DE)**

(72) Erfinder: **Haubrich, Gerhard, Dr. Dipl.-Chem., Am
Petrusacker, D-5300 Bonn 1(DE)**
Erfinder: **Prescher, Günter, Dr. Dipl.-Chem.,
Llesingstrasse 2, D-6450 Hanau 9(DE)**
Erfinder: **Faller, Jürgen, Alois-Ruppel-Strasse 3,
D-6459 Rodenbach(DE)**

**Beschreibung**

Die Erfindung betrifft ein Verfahren zur Herstellung von Tetrahydrofurfurylmethacrylat (THF-MA) durch Umesterung von Methyl- oder Ethylmethacrylat mit Tetrahydrofurfurylalkohol (Genaue Bezeichnung: Tetrahydrofurfuryl-(2)-Methylalkohol (THF-OH)). Die Herstellung von Acrylaten und Methacrylaten höherer Alkohole durch Umesterung von zum Beispiel Methylmethacrylat in Gegenwart von Alkoxiden ist aus der JP-PS 19716 (31.3.1975) bekannt.

Eine ähnliche Methode zur Herstellung der entsprechenden Dialkylaminoethylester betrifft die JP-PS 19911 (15.2.79).

Gemäß dieser Vorschrift soll man jedoch kein Alkalimetallalkoxid als Katalysator einsetzen.

C.E. Rehberg und W.A. Fanuette beschreiben die Herstellung und Eigenschaften von Acryl- und Methacrylsäureester Ethergruppen-haltiger Alkohole (J. Org. Chem. 14, 1094-8 (1949)).

Wegen der leichten Polymerisierbarkeit dieser Ester wird während der Reaktion eine Sauerstofffreie Atmosphäre vorgesehen.

THF-MA erhält man nach dieser in einer Ausbeute von 86%.

Bei allen Verfahren nach dem Stand der Technik wird der freigesetzte Alkohol zusammen mit kleinen Mengen des Ausgangsesters als Azeotrop abdestilliert, und so das Umesterungsgleichgewicht in die gewünschte Richtung verschoben. Die dazu notwendigen Temperaturen führen trotz aufwendiger Stabilisierungsmaßnahmen zu Ausbeuteverlusten aufgrund der hohen Polymerisationsneigung des Endprodukts. Es wäre wünschenswert, den oben beschriebenen Polymerisationsreaktionen wirksam Einhalt gebieten zu können. Da aber weitere Stabilisierungsmaßnahmen keine Wirkung zeigen, müssen die Reaktionsbedingungen schonender gestaltet werden.

Gegenstand der Erfindung ist ein Verfahren zur Herstellung von Tetrahydrofurfurylmethacrylat, dadurch gekennzeichnet, daß man Methyl- oder Ethylmethacrylat mit Tetrahydrofurfuryl-(2)-methylalkohol in Gegenwart des Tetrahydrofurfuryl-2-methoxids eines Alkali- oder Erdalkalimetalls umsetzt.

Vorzugsweise leitet man während der Umesterungsreaktion gleichzeitig getrocknete Luft oder reinen Sauerstoff durch das Reaktionsgemisch und erhält dann bessere Ausbeuten.

Die Luftmenge beläuft sich auf maximal 10 ml/h.l (Reaktionsvolumen).

Es empfiehlt sich die Umesterung in Gegenwart von nach dem Stand der Technik bekannten Stabilisatoren durchzuführen wie Hydrochinonen (beispielsweise Hydrochinonmonomethylether), Amine (Diphenylamin, Phenothiazin), Phenole (Topanol A).

Die Reaktionstemperatur liegt zwischen 45 und 130°C, vorzugsweise zwischen 60 und 80°C.

Als Katalysator setzt man das Tetrahydrofurfuryl-(2)-methoxid vorzugsweise des Lithium, Magnesium oder Calcium ein, das man entsprechend der ansonsten bekannten Herstellung von Alkoholaten durch Umsetzung von Li, CaH₂ oder Mg mit Furfurylalkohol gewinnt.

Man verwendet den Katalysator in einer Menge von 0,2 bis 10 Gew.-%, bevorzugt 1–3 Gew.-%, bezogen auf das Gesamtgewicht von eingesetztem Tetrahydrofurfurylalkohol und Methyl- beziehungsweise Ethylmethacrylat.

Die erfindungsgemäß verwendeten Katalysatoren beschleunigen die Reaktion außerordentlich stark und entfalten auch im Temperaturbereich zwischen 60 und 80°C ihre volle Wirksamkeit.

Man erzielt Raumzeitausbeuten von bis zu 2 Mol/h.l (bezogen auf das Volumen des Ansatzes).

Da die vorzugsweise eingestellten Temperaturen unterhalb des Siedepunkts beispielsweise des Methylmethacrylat/Methanol-Azeotrops liegen, arbeitet man bei Unterdrucken von bis zu 300 mbar.

Zu diesem Zweck bemißt man den durchgeleiteten Luftstrom natürlich so, daß das Vakuum nicht zusammenbricht.

Methyl- beziehungsweise Ethylmethacrylat und Tetrahydrofurfurylalkohol werden im molaren Verhältnis 1,4:1 bis 3,0:1, vorzugsweise 1,5 bis 2,0:1, eingesetzt.

Aufgrund der schonenden Bedingungen reduziert sich der Anteil an Nebenprodukten, bei denen es sich zum größten Teil um Polymerisate handelt, auf ein Minimum. Das bedeutet nicht nur, daß im Vergleich zum Stand der Technik weniger Ausgangsester verlorengeht, sondern auch, daß man bei extraktiver Aufarbeitung des Sumpfes eine hohe Produktreinheit erzielt, und daß man aufgrund der geringen Sumpfviskosität bei der destillativen Reinigung zu hohen Ausbeuten kommt.

Die hergestellte Verbindung eignet sich als Ausgangsverbindung für die Herstellung von Polymeren.

Beispiel 1

157 g THF-OH und 0,26 g Lithium werden bei 25–60°C solange gerührt, bis eine klare Lösung vorliegt. Man versetzt mit 255,3 g Methylmethacrylat (MMA) und 2,55 g Hydrochinonmonomethylether, liegt ein Vakuum von 500 mbar an und destilliert das entstehende MMA/MeOH-Azeotrop bei einer Kopftemperatur von ≤ 50°C ab. Bei einem Rücklaufverhältnis von etwa 10:1 ist dieser Vorgang nach 2–2,5 h beendet. Als zweite Fraktion wird nun bei 30–50 mbar überschüssiges MMA abgenommen und dann eventuell ein umgesetzter Alkohol als dritte Fraktion. Bis zu diesem Zeitpunkt werden geringe Mengen getrockneter Luft durch die Reaktionsmischung geleitet. Man legt nun ein Vakuum von 10 mbar an und nimmt bei einer Kopftemperatur von 98–100°C das Produkt ab. Ausbeute 231,4 g, Reinheit (GC) 96%.

Beispiel 2

Der Versuch wird wie in Beispiel 1 durchgeführt. Die erste Fraktion wird nun so abgenommen, daß die Sumpftemperatur nicht über 60°C steigt. Dazu wird das Vakuum in den Grenzen von 350–250 mbar variiert. Bei einem Rücklauf-Verhältnis von 10:1 ist die Abnahme des MMA/MeOH-Azeotrops nach etwa 3 h beendet. Man arbeitet auf wie in Beispiel 1. Ausbeute 238,2 g, Reinheit (GC) 96%

Beispiel 3

164 g THF-OH und 1,3 g Magnesium werden unter Rückfluß erhitzt, bis keine Wasserstoffentwicklung mehr zu erkennen ist. Man fügt 255,3 g MMA hinzu und 2,55 g Diphenylamin, legt ein Vakuum von 600 mbar an und destilliert das entstehende MMA/MeOH-Azeotrop bei einer Kopftemperatur von ≤ 53°C ab (R:E = 10:1). Weiteres Vorgehen wie in Beispiel 1 (Luftdurchleitung). Ausbeute 236 g, Reinheit (GC) 95%.

Beispiel 4

Man arbeitet wie in Beispiel 3, setzt jedoch 2,1 g CaH₂ statt 1,3 g Mg ein. Stabilisierung mit 2,55 g Topanol A (2,4-Dimethyl-6-tert.-butylphenol). Ausbeute 168 g, Reinheit (GC) 96%.

**Patentansprüche**

1. Verfahren zur Herstellung von Tetrahydrofurfuryl-(2)-methacrylat, dadurch gekennzeichnet, daß man Methyl- oder Ethylmethacrylat mit Tetrahydrofurfuryl-(2)-methylalkohol in Gegenwart des Tetrahydrofurfuryl-(2)-methoxids eines Alkali- oder Erdalkalimetalls umsetzt.

2. Verfahren gemäß Anspruch 1, dadurch gekennzeichnet, daß man während der Umsetzung durch das Gemisch getrocknete Luft oder Sauerstoff leitet.

3. Verfahren gemäß den Ansprüchen 1 und 2, dadurch gekennzeichnet, daß die Reaktion bei 45 bis 130°C, vorzugsweise zwischen 60 und 80°C, abläuft,

4. Verfahren gemäß den Ansprüchen 1 bis 3, dadurch gekennzeichnet, daß man das Methoxid des Li, Mg oder Ca verwendet.

5. Verfahren gemäß den Ansprüchen 1 bis 4, dadurch gekennzeichnet, daß man die Umesterung bei einem Druck von 300 bis 1013 mbar durchführt.

6. Tetrahydrofurfuryl-(2)methoxid eines Alkali- oder Erdalkalimetalls, insbesondere des Li, Mg oder Ca.

**Claims**

1. A process for the production of tetrahydrofurfuryl-(2)-methacrylate, characterized in that methyl or ethyl methacrylate is reacted with tetrahydrofurfuryl-(2)-methyl alcohol in the presence of the tetrahydrofurfuryl-(2)-methoxide of an alkali or alkaline-earth metal.

2. A process as claimed in claim 1, characterized in that air or oxygen is passed through the mixture during the reaction.

3. A process as claimed in claims 1 and 2, characterized in that the reaction takes place at 45 to 130°C and preferably at 60 to 80°C.

4. A process as claimed in claims 1 to 3, characterized in that the methoxide of Li, Mr or Ca is used.

5. A process as claimed in claims 1 to 4, characterized in that the transesterification is carried out under a pressure of 300 to 1013 mbar.

6. Tetrahydrofurfuryl-(2)-methoxide of an alkali or alkaline-earth metal, more especially Li, Mg or Ca.

**Revendications**

1. Procédé de préparation de tétrahydrofurfuryl-(2)-méthacrylate, caractérisé en ce qu'on fait réagir du méthyl- ou de l'éthylméthacrylate avec de l'alcool tétrahydrofurfuryl-(2)-méthylique en présence du tétrahydrofurfurylméthoxyde d'un métal alcalin ou alcalino-terreux.

2. Procédé selon la revendication 1, caractérisé en ce que, pendant la réaction, on fait barboter dans le mélange de l'air ou de l'oxygène secs.

3. Procédé selon les revendications 1 et 2, caractérisé en ce que la réaction a lieu de 45 à 130°C, de préférence entre 60 et 80°C.

4. Procédé selon les revendications 1 à 3, caractérisé en ce qu'on utilise le méthoxyde de Li, Mg ou Ca.

5. Procédé selon les revendications 1 à 4, caractérisé en ce qu'on réalise la transestérification sous une pression de 300 à 1013 mbar.

6. Tétrahydrofurfuryl-(2)-méthoxyde d'un métal alcalin ou alcalino-terreux, notamment de Li, Mg ou Ca.